(19) Europäisches Patentamt
European Patent Office
Office européen des brevets

(11) Publication number : 0 450 813 A2

# (12) EUROPEAN PATENT APPLICATION

(21) Application number : 91302460.0

(22) Date of filing : 21.03.91

(51) Int. Cl.$^5$ : C12N 15/00, C12N 15/12, C07K 15/00, C12P 21/02

The applicant has filed a statement in accordance with Rule 28 (4) EPC (issue of a sample only to an expert). Accession number(s) of the deposit(s): ATCC 68209.

(30) Priority : 23.03.90 US 498181

(43) Date of publication of application : 09.10.91 Bulletin 91/41

(84) Designated Contracting States : AT BE CH DE DK ES FR GB IT LI LU NL SE

(71) Applicant : MERCK & CO. INC.
126, East Lincoln Avenue P.O. Box 2000
Rahway New Jersey 07065-0900 (US)

(72) Inventor : Elbrecht, Alex
65 Maple Street
Watchung, NJ 07060 (US)
Inventor : Smith, Roy G.
528 Forest Avenue
Westfield, NJ 07090 (US)

(74) Representative : Thompson, John Dr. et al
Merck & Co., Inc. European Patent
Department Terlings Park Eastwick Road
Harlow, Essex CM20 2QR (GB)

## (54) Protein phosphatase inhibitor-1 gene and expression.

(57) The gene encoding the complete amino acid sequence of mammalian protein phosphatase inhibitor-1 (PPI-1) is disclosed. The gene is about 516 base pairs in length and the deduced amino acid sequence of the protein from rat has about 80% amino acid sequence homology with other mammalian species of protein phoshatase inhibitor-1. The novel gene is used to produce transgenic animals.

EP 0 450 813 A2

FIG 3

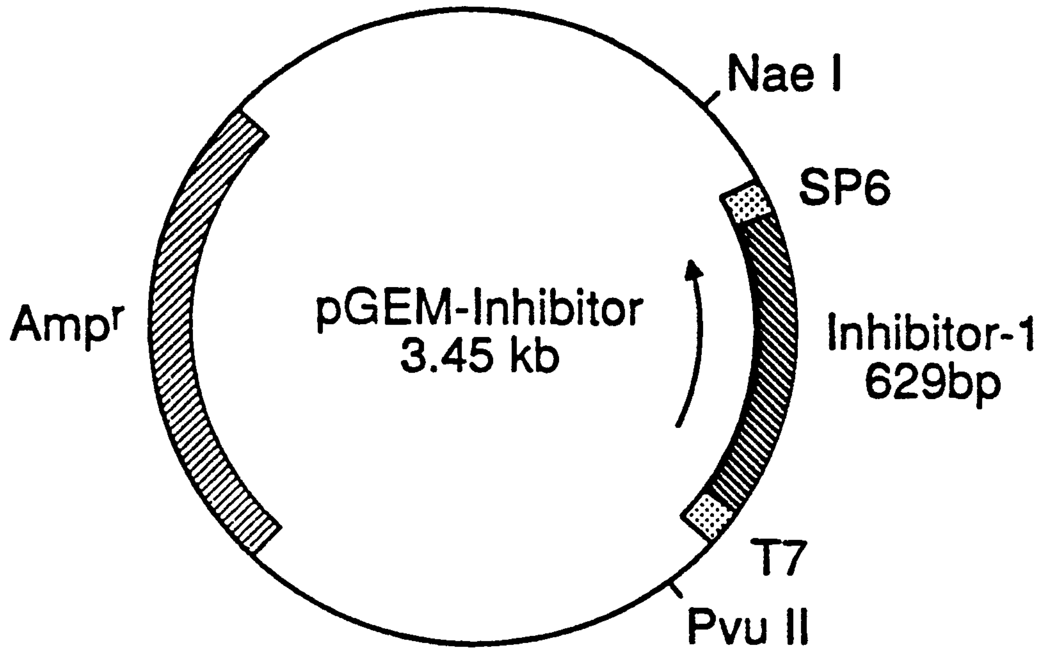

Nae I
SP6
Ampr
pGEM-Inhibitor
3.45 kb
Inhibitor-1
629bp
T7
Pvu II

# PROTEIN PHOSPHATASE INHIBITOR-1 GENE AND EXPRESSION

## BRIEF DESCRIPTION OF THE FIGURES

Figure 1. Nucleic acid sequence of protein phosphatase inhibitor-1 and deduced amino acid sequence.
Figure 2. This figure shows the pRSV-inhibitor vector.
Figure 3. This figure shows the pGEM-inhibitor vector.

## BACKGROUND OF THE INVENTION

Reversible protein phosphorylation plays a unique role in the control of processes essential for cell growth and viability. Modulation of the phosphorylation state of key regulatory proteins has been observed following stimulation with hormones and neurotransmitters. Generation of the intracellular second messenger cyclic AMP results in the increased phosphorylation of many target proteins by mechanisms resulting either directly or indirectly from the activation of cellular cyclic AMP-dependent protein kinases. The discovery of a potent thermostable protein inhibitor of the broad substrate specificity enzyme protein phosphatase-1 suggested an indirect route for increasing cellular protein phosphorylation (Shenolikar, FASEB, J. 2: 2753-2764 ,1988). Protein phosphatase inhibitor-1 is only active after it has been phosphorylated on a specific threonine residue by cyclic AMP-dependent protein kinase. In this manner, a substantial amplification of the cascade of protein kinase reactions can be achieved by the inhibition of protein phosphatase-1 (Cohen, Ann. Rev. Biochem. 58: 453-508, 1989). Cyclic AMP control can also be applied to pathways not directly subject to phosphorylation by cyclic AMP-dependent protein kinases.

Protein phosphatase-1 has been implicated as a key regulator of the eukaryotic cell cycle (Cyert and Thorner, Cell 57: 891-893, 1989). A gene isolated from the filamentous fungus Aspergillus nidulans ,bim G, encodes a protein that is about 86% homologous to the catalytic subunit of protein phosphatase-1 isolated from rabbit skeletal muscle (Doonan and Morris, Cell 57: 987-996, 1989; Cohen, FEBS Lett. 232: 17-23, 1988). While the loss of a functional bim G gene is not lethal, the protein phosphatase-1 is required for progression through mitosis (Doonan and Morris, Cell 57: 987-996, 1989). Genetic analyses also suggest a role for protein phosphatase-1 in the mitotic process in the fission yeast, Schizosaccharomyces pombe, (Ohkura et al., Cell 57: 997-1007, 1989; Booher and Beach, Cell 57: 1009-1016, 1989). Furthermore, microinjection of protein phosphatase inhibitor-1 in Xenopus oocytes inhibits meiosis suggesting that an active protein phosphatase-1 is necessary for meiotic maturation of the oocyte (Huchon et al., Nature 294: 358-359, 1981).

Protein phosphatase inhibitor-1 was first described as an inhibitor of phosphorylase phosphatase from rabbit skeletal muscle (Huang and Glinsmann, Eur. J. Biochem. 70: 419-426, 1976). The protein was subsequently purified from rabbit skeletal muscle (Nimmo and Cohen, Eur. J. Biochem. 87: 341-351, 1978) and its complete primary sequence determined (Aitken et al., Eur. J. Biochem. 126: 235-246, 1982). Protein phosphatase inhibitor-1 or inhibitor-1-like proteins have been characterized in several tissues (Cohen, Annu. Rev. Biochem. 58: 453-508, 1989). One such related protein, DARPP-32, is functionally similar but has only limited overall primary structural homology (less than 15% identity) with protein phosphatase inhibitor-1 (Williams et al., J. Biol. Chem. 261: 1890-1903, 1986). However, because nucleic acid probes and species specific antibodies have not been available direct evidence for the nature of the protein expressed in various species and cells has not been obtained. Indeed, the present invention provides probes for the investigation of protein phosphatase inhibitor-1 expression in different species and tissues, thus providing a performance advantage in food animals.

Cyclic AMP and phosphorylated PPI-1 resulting from β-adrenergic agonists (β-agonists) treatment play a significant role in the metabolism of glycogen. Indeed, β-agonists are potent growth promoters in many species of animals, Deshaies et al., Can. J. Physiol. Pharmacol. 59: 113-121 (1980); Emery et al., Biosci. Rep. 4: 83-91 (1984); Beermann et al., J. Anim Sci. 62: 370-380 (1986); and Hanrahan et al., in Recent Advances in Animal Nutrition, Haresigh, ed., pp. 125-138, Butterworth, London (1986). This group of compounds produces a large reduction in body fat content and a significant increase in skeletal muscle mass, Yang and McElliogott, Biochem. J. 261: 1-10 (1989).

Glycogen synthesis is increased initially by the binding of a β-agonist to a receptor on a muscle cell which initiates the formation of cyclic AMP. The cyclic AMP activates a protein kinase which in turn phosphorylates PPI-1, which is thereby activated. Thus, when glycogen degradation is switched on by cyclic AMP, the accompanying phosphorylation of PPI-1 keeps phosphorylase in the active a form. By both activating phosphorylase kinase and inhibiting phosphatase-1, rises in cyclic AMP levels have a much larger and sharper effect on glycogen synthesis and breakdown than if cyclic AMP regulated only one of these pathways. Consequently, animals made transgenic by introducing the gene for PPI-1, of the present invention, into germ cells should show enhanced growth due to the presence of greater amounts of PPI-1 or show greater sensitivity to the growth promoting effects of β-antagonists when used in much lower amounts. Since PPI-1 is most abundant in skeletal muscle and the majority of total phosphatase activity in skeletal muscle is due to phosphatase-1, the most significant effects on cellular hyprotrophy would be realized in skeletal muscle.

## OBJECTS OF THE INVENTION

It is accordingly, an object of the present invention to provide a recombinant DNA sequence for protein phosphatase inhibitor-1. A further object is to provide a recombinant gene capable of expressing protein phosphatase inhibitor-1. Another object is to provide a vector containing the DNA sequence for protein phosphatase inhibitor-1. A further object is to provide a host cell transformed with a vector containing the DNA sequence for protein phosphatase inhibitor-1. It is also an object to provide a recombinant process for making protein phosphatase inhibitor-1. It is a further object of the present invention to provide transgenic animals which contain in their germ line the gene for PPI-1.

## SUMMARY OF THE INVENTION

The gene encoding the complete amino acid sequence of mammalian protein phosphatase inhibitor-1 is disclosed. The gene is about 516 base pairs in length and the deduced amino acid sequence of the protein from rat has about 80% amino acid sequence similarity with other mammalian species of protein phosphatase inhibitor-1.

## DETAILED DESCRIPTION OF THE INVENTION

The present invention relates to the isolation, purification and characterization of the mammalian protein phosphatase inhibitor-1 (PPI-1) gene and related genetic information, incorporating the gene into an expression system, transforming prokaryotic and eukaryotic cells with the expression system and a method of producing PPI-1 employing the transformed cells. The gene and vectors containing the gene of the present invention are used to produce transgenic animals. The present invention also provides purified nucleic acid probes which will enable the determination of PPI-1 expression in various species and in various tissues and cells within the selected species.

The PPI-1 genetic information is obtained from either genomic DNA or mRNA. Genomic DNA is extracted from any tissue and prepared for cloning by techniques such as random fragmentation of high-molecular-weight DNA following the technique of Maniatis et al., Cell 15: 678-701 (1978) or by cleavage with restriction enzymes according to the method of Smithies et al., Science 202: 1248-1289 (1978). The isolated and purified genomic DNA is then incorporated into an appropriate cloning vector, Manitias, et al., Molecular Cloning, A Laboratory Manual, Cold Spring Harbor Laboratory, Cold Spring Harbor, New York (1982). Cellular RNA is isolated from tissue that expresses PPI-1, such as muscle, kidney, brain and the like using a technique such as that of Chirgwin et al., Biochem. 18: 5294-5299 (1979). The RNA is enriched for mNRA and cDNA is prepared by techniques known in the art. Double stranded cDNAs are cloned into appropriate vectors such as bacteriophage lambda gt10. Genomic and cDNA libraries consisting of approximately 1 x $10^6$ individual recombinants are generated and screened for the correct gene. Synthetic genes may be produced from known amino acid sequences of the PPI-1, such as the sequence described by Aitken et al., Eur. J. Biochem. 126: 235-246 (1982) for rabbit skeletal muscle PPI-1. The unique nucleotide sequence is derived from reverse translation of the amino acid sequence by techniques similar to that of Itakura et al., Science 198: 1056-1063 (1977). It is intended that nucleotide sequence for PPI-1 be interpreted to include all codons that code for the appropriate amino acid in the sequence for PPI-1, as indicated by the degeneracy of the genetic code. It is further intended that the nucleotide sequence for PPI-1 include truncated genes which encode biologically active fragments of PPI-1 such as, but not limited to, those described by Shenolikar et al., Biochem. Soc. Trans. 6: 935-937 (1978) and Aitken and Cohen, FEBS Letters 147: 54-58 (1982). Biologically active as used herein is the ability of the recombinant gene product to exhibit protein phosphatase inhibitor-1 activity which results in inhibition of protein phosphatase-1 resulting in a substantial amplification of the cascade of protein kinase reactions as discussed above. The recombinant gene product may be a protein or polypeptide that has fewer amino acids than the protein found in native tissue. In particular, the nucleotide base sequence that encodes the minimal active fragment of 46 amino acid residues from amino acid number 9 through 54 inclusive. The amino acid sequence of this fragment is identical in both the rabbit and rat, as indicated herein, and possesses full PPI-1 activity. This fragment also contains the phosphothreonine residue (position 35) necessary for activity. Further truncated forms expressed form the mutated gene sequences are also included in the scope of this invention.

The clonal genomic DNA and cDNA libraries are screened to identify the clones containing PPI-1 sequences by hybridization with an oligonucleotide probe. The sequence of the oligonucleotide hybridization probe may be based on the determined amino acid sequence of rabbit skeletal muscle PPI-1 and derived DNA or mRNA by procedures known in the art, such as,. Maniatis et al., supra; Anderson and Kingston, Proc. Natl. Acad. Sci. USA 80: 6838-6842 (1982) and Suggs et al., Proc. Natl. Acad. Sci. USA 78: 6613-6617 (1981).

The preferred process for obtaining a gene for mammalian PPI-1 is to prepare a cDNA library and screen for the appropriate genetic information. Total RNA is isolated from mammalian muscle, mammals include all non-human mammals with rat being preferred, using the method of Chirgwin et al., Biochem. 18: 5294-5299 (1979). A portion of the RNA is chromatographed on oligo(T)-cellulose (Pharmacia, Piscataway, NJ) to enrich for mRNA's according to the manufactures instructions. The isolated poly-$A^+$ RNA is used to prepare an oligo(T)-primed cDNA library using commercially available cDNA synthesis and cloning systems. Reverse transcriptase is used to synthesize and label the first DNA strand with [alpha-$^{32}$P]dCTP. Following removal of the RNA with E. coli ribonuclease H, the second DNA strand is synthesized and labeled like the first strand. Aliquots of the first and second strand reactions are subjected to electrophoresis through denaturing (alkaline) agarose gels. Autoradiography of the dried gels containing the radiolabelled DNA strands reveal that both the first and second strands yield products greater than 1.0 kilobase (Kb) in length.

The double-stranded cDNAs are inserted into cloning vectors. Cloning vector as used herein is defined as a DNA molecule, usually a small plasmid or bacteriophage DNA capable of self-replication in a host organism, and used to introduce a fragment of foreign DNA into a host cell. The foreign DNA combined with the vector DNA constitutes a recombinant DNA molecule which is derived form recombinant technology. Cloning vectors may include plasmids, bacteriophage, viruses and cosmids. It is to be understood that any cloning vector may be used to clone the novel PPI-1 DNA sequence, with the lambda gt10 vector being preferred. Prior to ligation of the PPI-1 DNA into lambda gt10, the EcoRI sites are methylated with EcoRI methylase, see Davis et al., Basic Methods In Molecular Biology, Elsevier, N. Y., pp. 32-33, 1986. EcoRI linkers are added and the cDNAs are ligated into lambda gt10 vector arms. A cDNA library consisting or approximately 1 x $10^6$ independent recombinant phage clones is generated.

The cDNA library is screened with a synthetic oligonucleotide probe containing about 60 base pairs (bp) encoding amino acids about 8 to about 27 in the rabbit PPI-1 primary sequence (Aitken et al., Eur. J. Biochem. 126: 235-246 [1982]). The oligonucleotide sequence is generated using the rabbit codon bias usage and is shown in the following table.

TABLE 1

Screening Probe Oligonucleotide Sequence

5' - CTCGGCGGCC TCGGGGTCCA GGTGGGGCTC
CAGCAGGGGC ACGGTGAACT GGATCTTCCC -
3'

Southern DNA hybridization analyses with genomic DNA from GH3 rat pituitary cells are performed to determine conditions suitable for the detection of specific cross-hybridizable bands. Hybridization is carried out on nitrocellulose filters overnight at about 42° C in about 35% formamide, about 4 x SSC, about 0.1 M sodium phosphate, about pH 6.5, about 1 x Denhardt's solution, about 0.1% sodium pyrophosphate, about 0.1% sodium dodecyl sulphate (SDS), about 0.1 mg/ml herring sperm DNA, and about 10% dextran sulphate. The filters are washed for about 1 h at about room temperature in 2 x SSC containing 0.5% SDS, followed by about 1 h at 50° C in 1 x SSC containing about 0.5% SDS. Identical conditions and procedures are used to screen the PPI-1 cDNA library.

A total of 1 x $10^5$ plaques are plated for the primary screen. Five positive clones are identified and 4 are plaque purified through secondary and tertiary screens. The DNA from these individually purified plaques readily hybridize to the synthetic oligonucleotide used in the screening. The Eco R1 inserts are excised and subcloned into the phagemid pUC 120 following the procedures of Viera, Ph.D. Thesis, University of Minnesota, 1989. Three of these clones, designated pPPI-1, pPPI-2 and pPPI-10 are extensively characterized by restriction enzyme analysis and DNA sequencing (Sequenase, United States Biochemical Corp. Cleveland, OH). Clone pPPI-10 is present in the 5′ to 3′ orientation and has been sequenced in its entirety. The nucleotide base sequence and the deduced amino acid sequence of the pPPI-10 clone is shown in Figure 1. The DNA contains a coding region of about 516 nucleotides flanked by about 6 bases of 5′-untranslated sequence and about 97-bases of 3'-untranslated sequence. Translation of this open reading frame yields a polypeptide of about 171 amino acids. Alignment of the deduced amino acid sequence with the 166 amino acid primary sequence for rabbit PPI-1 inhibitor indicated an overall sequence identity of about 80%, Aitken et al., Eur. J. Biochem. 126: 235-246 (1982).

The recombinant DNA from a representative clone, pPPI-10 contained in a host Escherichia coli has been deposited under the Budapest Treaty in the American Type Culture Collection, 12301 Parklawn Drive, Rockville, Maryland 20852, USA. Deposit was made on January 25, 1990 and the culture was given an ATCC designation of 68209.

Translation of the mRNA encoded by the gene for PPI-1 is carried out in Xenopus oocytes. The PPI-1 gene is incorporated into the pGEM (Promega) to produce the vector pGEM-inhibitor (Figure 3). This vector makes use of the SP6 and T7 RNA polymerases to synthesize PPI-1 RNA in vitro. The pGEM-2 vector is digested with Hind III and Sac I endonucleases then treated with T4 DNA polymerase to remove the 3'-"overhang" from the Sac I site and fill in the Hind III site. Since the ends are now flush the vector is religated. This process is performed to remove the restriction enzyme sites in the poly-linker with the exception of the Eco RI site.The recombinant PPI-1 DNA insert (bracketed by Eco RI sites) form the vector pPPI-10 is ligated into the modified pGEM vector. Orientation (5' to 3') is designated by the arrow in Figure 3. All protocols are adequately described by materials supplied by the vendor (Pormega). Adult female Xenopus laevis are anesthetized and the oocytes removed. The oocytes are cleaned by agitation for about 3 h in about 2 mg/ml collagenase in Ca-free saline following the procedure of Leonard et al., J. Neuroscience 7: 875-881 (1987). After digestion, stage V and VI oocytes are injected with about
1-5 ng samples of the in vitro synthesized RNA following the technique of Leonard. Vehicle injected oocytes served as controls. Protein phosphate inhibitor-1 can be identified in cultured cells.

Expression of the recombinant DNA encoding PPI-1 is accomplished in a number of different host cells with a variety of expression vectors. Expression vectors are defined herein as DNA sequences that are required for the transcription of cloned copies of recombinant DNA sequences or genes and the translation of their mRNAs in an appropriate host. Such vectors can be used to express eukaryotic genes in a variety of hosts such as bacteria, blue-green algae, yeast cells, insect cells, plant cells and animal cells. The PPI-1 may also be expressed in a number of virus systems. Specifically designed vectors allow the shuttling of DNA between bacteria-yeast, bacteria-plant or bacteria-animal cells. An appropriately constructed expression vector should contain: an origin of replication for autonomous replication in host cells, selective markers, a limited number of useful restriction enzyme sites, a high copy number, and strong promoters. A promoter is defined as a DNA sequence that directs RNA polymerase to bind to DNA and to initiate RNA synthesis. A strong promoter is one which causes mRNAs to be initiated at high frequency. Expression vectors may include, but are not limited to, cloning vectors, modified cloning vectors, specifically designed plasmids or viruses. Expression vectors which can be used for the expression of PPI-1 include, but are not limited to, pBR322, ptac12, ptrpL1, pSA1, pRSV (see Fig. 2) and pGEM (see Fig. 3). The specifically selected use of the PPI-1 DNA (gene) will determine the appropriate vector for expression as described above and below.

The recombinant PPI-1 DNA and/or subunits thereof are used as probes to examine the expression of PPI-1 mRNA in various tissues of different animal species by northern blot analysis. Total cellular RNA is prepared by the guanidinium isothiocyanate/CsCl method as described above. The RNA is separated on agarose (1.2 % w/v) formaldehyde denaturing gels by the method of Maniatis et al., (supra) and transferred to nitrocellulose filters. Filters are prehybridized and hybridized as described by Thomas, Proc. Natl. Acad. Sci. USA 79: 5201-5205 (1980). The $^{32}$P-labelled hybridization probe is generated using a random oligonucleotide priming kit (Boehringer Mannheim) in the presence of $^{32}$P-dCTP using either plasmid pPPI-10 or the Eco RI insert purified by electrophoresis on about 1 % (w/v) low-melt agarose gels as the template. Protein phosphatase inhibitor-1 mRNA is readily detected in rat skeletal muscle, brain and kidney using total cytoplasmic RNA form these tissues. Inhibitor-1 mRNA is also present in rabbit skeletal muscle, brain and kidney and unlike the rat can be identified in the liver.

The PPI-1 recombinant DNA is also used to produce transgenic animals which can express the protein at levels higher than non-transgenic animals. Animals as used herein includes non-human mammals, birds and fish. The expression vectors, described below, will allow the enhanced expression to selectively take place in muscle tissue which results in enhanced growth of the muscle tissue. The recombinant PPI-1 gene is incorporated into cells so that the gene will be part of the genome of germ cells, such as the egg and the sperm or primordial germ cells. Offspring of an animal containing the gene will also contain the gene and will show muscle growth characteristics induced by the expression of the PPI-1 gene.

The recombinant PPI-1 gene is used to transform animal ova, sperm, germ cells or zygotes by techniques known in the art which include, but are not limited to, microinjection , viruses and liposomes. The prefered process for germ cell incorporation is to introduce the PPI-1 gene into an animal zygote by procedures and techniques disclosed by Brinster et al., Proc. Natl. Acad. Sci. USA 82: 4438-4442 (1985) and in U.S. Patent 4,873,191. The recombinant PPI-1 DNA, as described above and in Example 1, is inserted into an appropriate expression vector which is used to transform the germ cells and somatic tissues. The gene for PPI-1 is incorporated into a modification of the pRSV luciferase expression vector construct described by de Wet et al., Mol. Cell. Biol. 7: 725-737 (1987). This vector makes use of the Rous sarcoma virus long terminal repeat to drive expression of the PPI-1 recombinant DNA to muscle tissues. The pRSV inhibitor vector is prepared by digesting the pRSV luciferase vector with Hind III and Sma I endonucleoases to remove the luciferase gene. Plasmid PPI-10, described above and in Example 1, is digested with Hind III (no sites in PPI-1 recombinant DNA) and Dra 1 (one site in PPI-1 recombinant DNA at position 598). The 3′ end of the PPI-1 DNA is truncated at about position 598 and the 5′ end has the polylinker (from Eco RI to Hind III) form the pUC 120 vector attached. The PPI-1 fragment is ligated into the RSV-based vector regenerating the Hind III site and destroying both the Sma I and the Dra I endonuclease sites. The resultant vector contains a unique Nde I site at about position 2297 of the original pBR 322 vector, Suteliffe et al., Proc. Natl. Acad. Sci. USA 75: 3737-3741 (1978), and a unique Apa 1 site in the SV40 portion at position 2258, Reddy et al., Science 200: 494-502 (1978), Fiers et al., Nature 273: 113-120 (1978) and Fiers et al., Eur. J. Biochem. 122: 51-60 (1979). The pRSV inhibitor vector is used to transform Escherichia coli with the transformed cells being used to propagate the plasmids.

The gene can also be incorporated into plasmids containing other promoters and enhancer elements that direct expression of the gene in skeletal muscle. Other skeletal muscle directed promoters and enhancer elements include, but are not limited to, alpha-skeletal actin and muscle creatine kinase promoter and enhancer elements. The alpha-skeletal actin (ASA) promotor can be substituted for the RSV promoter in the above described vector. The ASA fragment contains the transcription start site and 5′-flanking promoter region (Bergsma et al., Mol. Cell. Biol. 6: 2462-2475, 1986). The ASA promoter directs expression of selected genes to skeletal muscle in transgenic mice (DiLella et al., Nucl. Acids. Res. 16: 4159,1988). Similarly specific muscle creatine kinase sequence elements which have been shown to direct expression to skeletal inuscle in transgenic mice (Johnson et al., Mol. Cell. Biol. 9: 3393-3399, 1989) can also be used to express the recoinbinant PPI-1 gene.

The plasmids are isolated from the bacterial cultures by procedures well known in the art. Linear DNA fragments are prepared by digestion with Nde I and Apa 1 endonucleoases and the PPI-1 recombinant DNA fragment is purified. The DNA concentration is determined by measuring the flourescence of bisbenzimide H3358, Labarca and Paigen, Anal. Biochem. 102: 344-352 (1980). The PPI-1 DNA is diluted to about 1-2 μg/ml which results in a few hundred copies of the foreign DNA per injected egg. The injection of the DNA is made through the zona pellucida and vitellus into one of the pronuclei, preferrably the male pronuclus, as described by Brinster et al., Proc. Natl. Acad. Sci. USA 82: 4438-4442 (1985). After injection the eggs are washed and transferred to the oviducts of pseudopregnant recipient animals. The presence of PPI-1 DNA is determined by the procedure described by Brinster et al.

The following examples illustrate the present invention without, however, limiting the same thereto.

## EXAMPLE 1

### Preparation Of Rat Muscle cDNA Library

Total cellular RNA was isolated form gastrocnemius muscle of male Sprague-Dawley rats (Charles River) using the guanidinium isothiocyanate/CsCl procedure of Chirgwin, et al., Biochemistry 18: 5294-5299 (1979). Approximately 15 mg of total RNA was obtained form 36 g of muscle tissue. A portion of the RNA (6.4 mg) was chromatographed on oligo(dT)-cellulose (Pharmacia) to enrich for mRNAs according to the manufacturer's instructions. Poly A+ RNA (5 μg) was used to prepare an oligo(dT)-primed cDNA library using commercially available cDNA synthesis and cloning systems. Reverse transcriptase was used to synthesize and label (with [alpha-$^{32}$P]dCTP) the first DNA strand. After removal of the RNA with E.coli ribonuclease H, the second DNA strand was synthesized and also labelled with [alpha-$^{32}$P]dCTP. Aliquots from each reaction were subjected to electrophoresis through denaturing (alkaline) agarose gels by procedures known in the art. Autoradiography of the dried gel containing radiolabelled DNA strands was used to obtain an approximation of the sizes of the newly synthesized first and second strands. Both strands on average were greater than 1 Kb in length.

Prior to ligation into lambda gt10 phage, the Eco RI sites were methylated with Eco RI methylase. Eco RI linkers were added and the cDNAs were ligated into lambda gt10 vector arms. The resultant lambda library contained greater than 1 x $10^6$ individual recombinants and was used to clone protein phosphatase inhibitor-1. Materials and methods for the above processes are either commercially available from Amersham or described by Maniatis et al., supra.

The skeletal muscle cDNA library was screened with a 60 bp synthetic oligonucleotide encoding amino acids 8-27 in the rabbit inhibitor-1 primary sequence as described by Aitken et al., Eur. J. Biochem. 126: 235-246 (1982), and shown in Table 1. The oligodeoxynucleotide sequence was generated by using the rabbit codon bias usage. Southern DNA hybridization analysis with genomic DNA from GH3 rat pituitary cells were performed to determine conditions suitable for detection of specific cross-hybridizable bands. Hybridization of the DNA on the nitrocellulose filters was carried out overnight at 42° C in 35 % formamide, 4 x SSC, 0.1 M sodium phosphate, pH 6.5, 1 x Denhardt's solution, 0.1 % sodium pyrophosphate, 0.1 % sodium dodecyl sulfate (SDS), 0.1 mg/ml herring sperm DNA, and 10 % dextran sulphate. The filters were washed for 1 h at room temperature in 2 x SSC containing 0.5 % SDS, followed by 1 hr at 50° C in 1 x SSC containing 0.5 % SDS. Identical conditions for hybridization and washing of nitrocellulose filters were used during the screening of the PPI-1 cDNA library. A total of 1 x $10^5$ plaques were plated for the primary screen. Five positive clones were identified and four were plaque purified through secondary and tertiary screens. The Eco RI inserts were excised, subcloned into the phagemid pUC 120 (Viera, Ph.D. Thesis, University of Minnesota [1989]) and characterized by restriction enzyme analysis and DNA sequencing (Sequanase, United States Biochemical Corp).

Three of the plaque purified clones, termed pPPI-1, pPPI-2 and pPPI-10, were extensively characterized by restriction enzyme analysis and DNA sequencing. The clone pPPI-10 was present in the 5′ to 3′ orientation and was sequenced in its entirety (Figure 1). A coding region 516 nucleotides long flanked by 6 bp of 5′-untranslated sequence and 97-bp of 3′-untranslated sequence was observed. Translation of this open reading frame yielded a 171 amino acid polypeptide. Alignment of the deduced polypeptide with the 166 amino acid primary sequence for rabbit PPI-1 indicated an overall identity of 80 %.

## EXAMPLE 2

### PPI-1 Expression Vectors

The pRSV inhibitor vector was made by digesting the vector pRSV luciferase with Hind III and Sma I to remove the luciferase gene, De Wet et al., Mol. Cell. Biol. 7: 725-737 (1987). The plasmid pPPI-10 from Example 1 was digested with Hind III and Dra 1. There are no Hind III sites and only one Dra 1 site within the PPI-1 recombinant DNA. The 3′ end of the PPI-1 DNA was truncated at position 598 and the 5′ end has the polylinker (from Eco RI to Hind III) from the pUC 120 vector attached. The PPI-1 fragment was ligated into the RSV-based vector regenerating the Hind III site and destroying both the Sma I and the Dra I endonuclease sites, Figure 2. The resultant vector contains a unique Nde I site at position 2297 of the original pBR 322 vector, Sutcliffe, Proc. Natl. Acad. Sci. USA 75: 3737-3741 (1978), and a unique Apa 1 site in the SV40 potion at position 2258, Reddy et al., Science 200: 494-502 (1978), Fiers et al., Nature 273: 113-120 (1978) and Fiers et al., Eur. J. Biochem. 122: 51-60 (1979). These unique restriction sites are used to make the PPI-1 gene linear for the production of transgenic animals.

The PPI-1 recombinant DNA was also inserted into the pGEM vector for the production of mRNA for in vitro protein synthesis. A sample of pGEM-2 (Promega) was digested with Hind III and Sac I. The digested vector was treated with T4 DNA polymerase to remove the 3′-overhang following Sac I digestion and to fill in the Hind III site and the vector was religated. This process was performed to remove the restriction enzyme sites in the poly-linker with the exception of the Eco RI site. The recombinant DNA insert of PPI-1 (bracketed by Eco RI sites) from the vector pPPI-10 was ligated into the modified pGEM vector. Orientation (5′ to 3′) is demonstrated by the arrow in Figure 3.

## EXAMPLE 3

### Northern RNA Hybridization Analysis Of Rat And Rabbit Tissues

Total cellular RNA was prepared from various tissues of male Sprague-Dawley rats and New Zealand White rabbits using the process described in Example 1. The RNA was subjected to electrophoresis through 1.2 % (w/v) agarose formaldehyde denaturing gels by the process of Maniatis et al., (supra), and transferred to nitrocellulose filters. Filters were prehybridized and hybridized as described by Thomas, Proc. Natl. Acad. Sci. USA 79: 5201-5205 (1980). The $^{32}$P-labelled hybridization probe was generated using a random oligonucleotide priming kit (Boehringer Mannheim) in the presence of 32P-dCTP using either plasmid pPPI-10 or the Eco RI insert purified by electrophoresis on 1 % (w/v) low-melt agarose gels as the template. Inhibitor-1 mRNA was readily detected in rat skeletal muscle, brain and kidney, using total cytoplasmic RNA from these tissues. Two distinct bands were observed. The major band, PPI-1, was estimated to be approximately 700 nucleotides in length while the minor species was estimated to be approximately 1.8 Kb in length. The 1.8 Kb species appears to be an alternate inhibitor-1 mRNA since it is only observed in tissues also expressing the 700 nucleotide species and the ratio of the two mRNAs appear approximately constant. Under the conditions used, PPI-1 mNRA was not detected in rat heart, lung or liver. Using poly-$A^+$-RNA (10 µg), a faint signal was detected in rat heart under conditions where no liver mRNA could be detected.

Similarly, the highest level of PPI-1 mRNA expression in rabbit was seen in skeletal muscle. However, the expression of the 0.7 Kb mRNA was detected, albeit faintly, using the total rabbit liver RNA. The poly-$A^+$-RNA from rabbit liver confirmed the presence of PPI-1 mRNA. This was estimated to be at least 10-fold lower than that seen in rabbit skeletal muscle.

## Claims

1. A purified and isolated DNA sequence encoding for biologically active protein phosphatase inhibitor-1.

2. The DNA sequence of claim 1 wherein said DNA sequence is truncated and encodes for biologically active protein phosphatase inhibitor-1.

3. The DNA sequence of claim 1 or claim 2 wherein said sequence is a recombinant DNA.

4. The DNA sequence of claim 1 wherein the DNA is obtained from rat tissue.

5. A recombinant DNA sequence comprising at least the following nucleotide sequence:

GGCGCC ATG GAG CCC GAC AAC AGT CCA CGG AAG ATC 39
CAG TTT ACG GTT CCG CTG CTG GAG CCT CAC CTG 69
GAC CCG GAG GCA GCC GAG CAG ATT CGG AGG CGC 102
CGC CCC ACC CCT GCC ACA CTT GTG CTG ACC AGC 135
GAC CAG TCA TCC CCA GAA GTA GAT GAA GAC CGG 168
ATC CCC AAC CCA CTT CTC AAG TCC ACA CTG TCA 201
ATG TCT CCA CGG CAA CGG AAG AAG ATG ACA AGG 234
ACT ACA CCC ACC ATG AAA GAG CTC CAG ACA ATG 267
GTT GAA CAT CAC CTA GGG CAA CAG AAA CAA GGG 300
GAA GAA CCT GAG GGA GCC ACT GAG AGC ACA GGG 333
AAC CAG GAG TCC TGC CCA CCT GGG ATC CCA GAC 366
ACA GGC TCA GCG TCA AGG CCA GAT ACC TCG GGG 399
ACA GCA CAA AAG CCT GCA GAA TCC AAA CCC AAG 432
ACT CAG GAG CAG CGT GGT GTG GAG CCC AGC ACA 465
GAG GAC CTT TCA GCC CAC ATG CTA CCA CTG GAT 498
TCC CAA GGA GCC AGC TTG GTC TGA CAGAAGTTGA 532
CATCCGGGGA TCGCCAGTGA GTGTGGAAGT 562
TCATGGACAC TGGATGTTTC TTAATCTCTT 592
GTTTTTAAAC GTGATAAATT TGGTGTTAAA 622
AAAAAAA 629

and truncated forms which encode biologically active protein phosphate inhibitor-1.

6. A recombinant rat protein phosphatase inhibitor-1 protein comprising at least the following amino acid sequence:

5 10
Met Glu Pro Asp Asn Ser Pro Arg Lys Ile Gln Phe Thr Val

15 20 25
Pro Leu Ley Glu Pro His Leu Asp Pro Glu Ala Ala Glu Gln

30 35 40
Ile Arg Arg Arg Arg Pro Thr Pro Ala Thr Leu Vla Leu Thr

45 50 55
Ser Asp Gln Ser Ser Pro Glu Val Asp Glu Asp Arg Ile Pro

60 65 70
Asn Pro Leu Leu Lys Ser Thr Leu Ser Met Ser Pro Arg Gln

75 80
Arg Lys Lys Met Thr Arg Thr Thr Pro Thr Met Lys Glu Leu

85 90 95
Gln Thr Met Val Glu His His Leu Gly Gln Gln Lys Gln Gly

100 105 110
Glu Glu Pro Glu Gly Ala Thr Glu Ser Thr Gly Asn Gln Glu

115 120 125
Ser Cys Pro Pro Gly Ile Pro Asp Thr Gly Ser Ala Ser Arg

130 135 140
Pro Asp Thr Ser Gly Thr Ala Gln Lys Pro Ala Glu SerLys

145 150
Pro Lys Thr Gln Glu Gln Arg Gly Val Glu Pro Ser Thr Glu

155 160 165
Asp Leu Ser Ala His Met Leu Pro Leu Asp Ser Gln Gly Ala

171
Ser Leu Val

and biologically active truncated forms thereof.

7. A vector containing the DNA sequence of any one of claims 1, 2 and 5.

8. A host cell transformed by the vector of claim 7 containing the DNA sequence coding for protein phosphatase inhibitor-1.

9. A process for the preparation of protein phosphatase inhibitor-1 comprising culturing the transformed host cell of claim 8 under conditions suitable for the expression of protein phosphatase inhibitor-1 and recovering protein phosphatase inhibitor-1.

10. Protein phosphatase inhibitor-1 made by the process of claim 9.

11. A transgenic animal in which germ cells and somatic cells contain a recombinant DNA sequence which encodes protein phosphatase inhibitor-1 in skeletal muscle resulting in skeletal muscle hypertrophy.

FIG 1

5 10
Met Glu Pro Asp Asn Ser Pro Arg Lys Ile
GGCGCC ATG GAG CCC GAC AAC AGT CCA CGG AAG ATC 39

15 20
Gln Phe Thr Val Pro Leu Leu Glu Pro His Leu 69
CAG TTT ACG GTT CCG CTG CTG GAG CCT CAC CTG

25 30
Asp Pro Glu Ala Ala Glu Gln Ile Arg Arg Arg
GAC CCG GAG GCA GCC GAG CAG ATT CGG AGG CGC 102

35 40
Arg Pro Thr Pro Ala Thr Leu Vla Leu Thr Ser
CGC CCC ACC CCT GCC ACA CTT GTG CTG ACC AGC 135

45 50
Asp Gln Ser Ser Pro Glu Val Asp Glu Asp Arg
GAC CAG TCA TCC CCA GAA GTA GAT GAA GAC CGG 168

55 60 65
Ile Pro Asn Pro Leu Leu Lys Ser Thr Leu Ser
ATC CCC AAC CCA CTT CTC AAG TCC ACA CTG TCA 201

70 75
Met Ser Pro Arg Gln Arg Lys Lys Met Thr Arg
ATG TCT CCA CGG CAA CGG AAG AAG ATG ACA AGG 234

80 85
Thr Thr Pro Thr Met Lys Glu Leu Gln Thr Met
ACT ACA CCC ACC ATG AAA GAG CTC CAG ACA ATG 267

90 95
Val Glu His His Leu Gly Gln Gln Lys Gln Gly
GTT GAA CAT CAC CTA GGG CAA CAG AAA CAA GGG 300

100 105
Glu Glu Pro Glu Gly Ala Thr Glu Ser Thr Gly
GAA GAA CCT GAG GGA GCC ACT GAG AGC ACA GGG 333

FIG 1 (cont)

```
110                            115                               120
Asn Gln Glu Ser  Cys Pro  Pro  Gly  Ile  Pro  Asp
AAC CAG GAG TCC  TGC CCA  CCT  GGG  ATC  CCA  GAC   366

                      125                      130
Thr Gly  Ser Ala  Ser  Arg  Pro  Asp  Thr  Ser  Gly
ACA GGC  TCA GCG  TCA  AGG  CCA  GAT  ACC  TCG  GGG   399

                135                      140
Thr Ala  Gln Lys  Pro  Ala  Glu  Ser  Lys  Pro  Lys
ACA GCA  CAA AAG  CCT  GCA  GAA  TCC  AAA  CCC  AAG   432

          145                  150
Thr Gln  Glu Gln  Arg  Gly  Val  Glu  Pro  Ser  Thr
ACT CAG  GAG CAG  CGT  GGT  GTG  GAG  CCC  AGC  ACA   465

     155                      160
Glu Asp  Leu Ser  Ala  His  Met  Leu  Pro  Leu  Asp
GAG GAC  CTT TCA  GCC  CAC  ATG  CTA  CCA  CTG  GAT   498

165                      170
Ser Gln  Gly Ala  Ser  Leu  Val
TCC CAA  GGA GCC  AGC  TTG  GTC  TGA  CAGAAGTTGA      532

CATCCGGGGA  TCGCCAGTGA  GTGTGGAAGT                    562

TCATGGACAC  TGGATGTTTC  TTAATCTCTT                    592

GTTTTTAAAC  GTGATAAATT  TGGTGTTAAA                    622

AAAAAAA                                               629
```

FIG 2

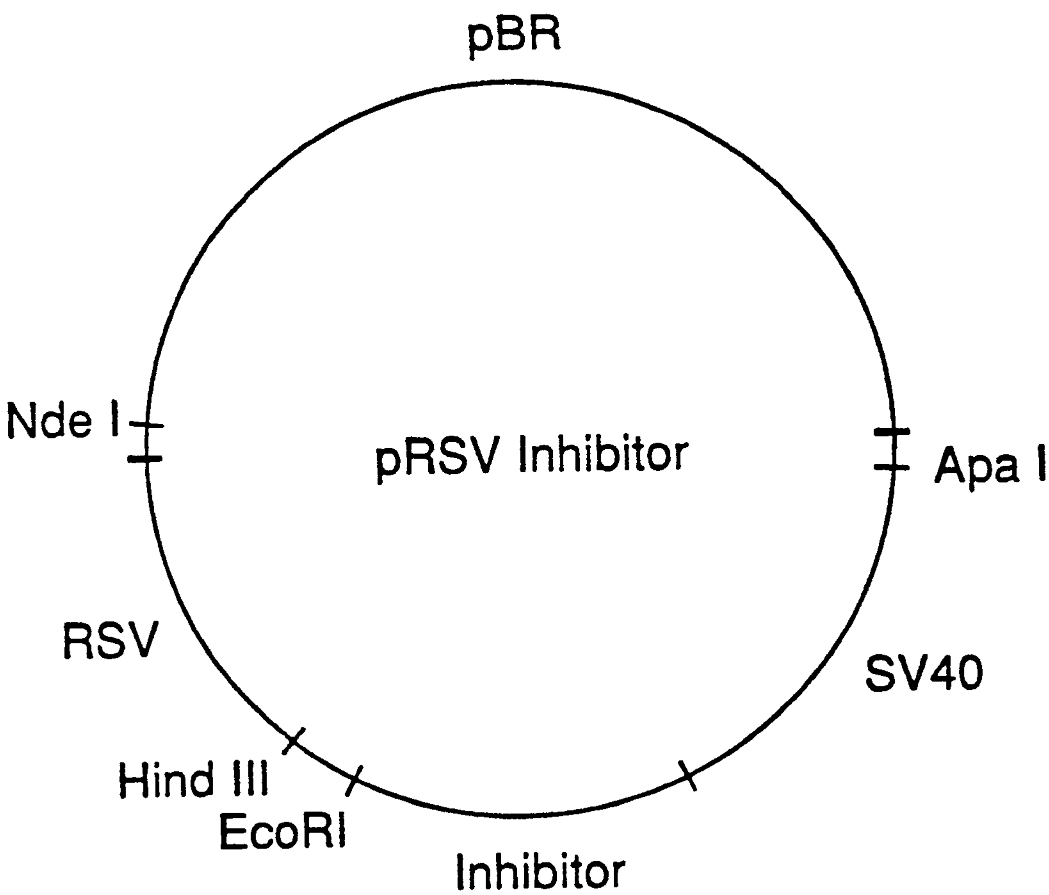
pBR
Nde I
pRSV Inhibitor
Apa I
RSV
SV40
Hind III
EcoRI
Inhibitor

FIG 3

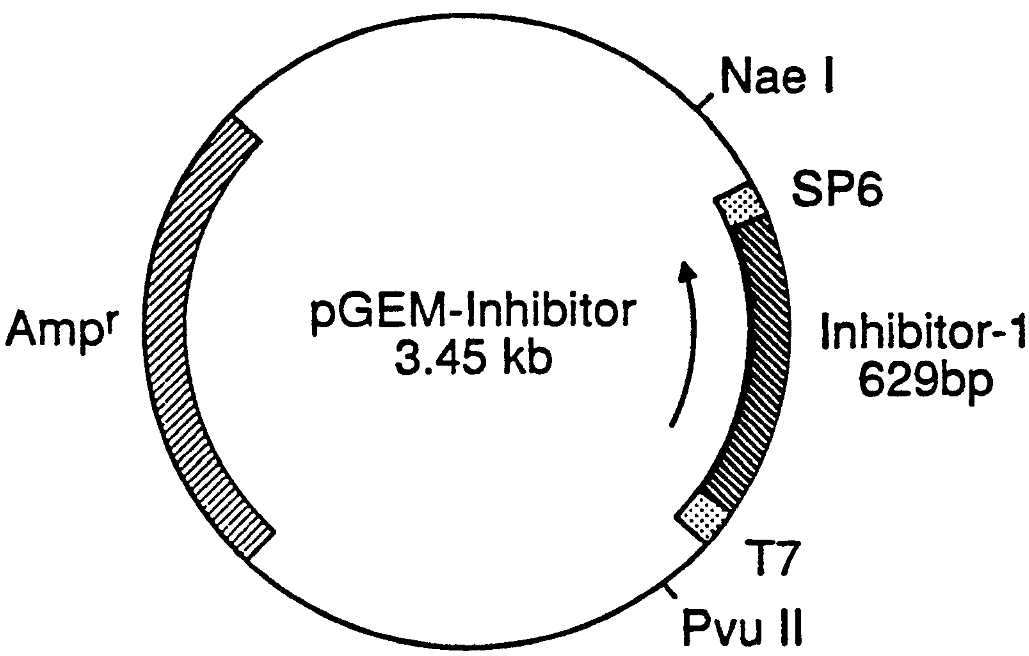
Nae I
SP6
Ampr
pGEM-Inhibitor
3.45 kb
Inhibitor-1
629bp
T7
Pvu II